# EUROPEAN PATENT APPLICATION

(11) **EP 1 355 153 A1**
(43) Date of publication of application: **22.10.2003**
(21) Application number: 02008844.9
(22) Date of filing: 19.04.2002
(51) Int. Cl.: G01N 33/50

(54) **Screening method for agents against cardiovascular diseases**

(71) Applicant: NAMAXX GENOMICS GMBH, 70569 Stuttgart (DE)
(72) Inventor: Villatte, Francois, Dr., 70569 Stuttgart (DE); Bachmann, Till. T., Dr., 70569 Stuttgart (DE)
(74) Representative: Becker Kurig Straus

(57) **Abstract**

The present invention relates to a method to identify potential drugs for treating or preventing diseases of the cardiovascular system in humans and animals, which method comprises culturing a living avian embryo; incubating said avian embryo with at least one first compound or composition, said at least one first compound or composition causing a detectable change in the activity of the cardiovascular system of said avian embryo; incubating said avian embryo with at least one second compound or composition, said at least one second compound or composition being to be tested as a potential drug; and detecting and/or monitoring an effect of said at least one second compound or composition and/or said at least one first compound or composition on the cardiovascular system of said bird embryo.

## Description

The present invention relates to a method to identify potential drugs for treating and/or preventing diseases of the cardiovascular system in humans and animals. In particular, the present invention pertains to a screening method, wherein avian embryos are utilized as a biological model for searching for compounds active against heart failure.

Heart failure, and in particular Congestive Heart Failure (CHF), is one of the major causes of combined morbidity and mortality in industrialized nations. Congestive Heart Failure occurs when the heart is damaged from diseases such as e.g. high blood pressure, heart attack or arteriosclerosis and is characterized by a reduced contraction and delayed relaxation of the heart. The failing, inefficient heart eventually results in fluid retention and shortness of breath, fatigue and exercise intolerance.

The current treatment of CHF is mainly directed to reduce the heart's workload by rest, by controlling sodium and water retention by means of a low sodium diet or by administering diuretics. Also the heart's activity has been tried to be influenced by administering inotropic agents, such as digoxin or vasodilators such as captopril.

So far several agents have been devised to treat heart failure.

In this respect EP 0 951 906 discloses the Corticotropin Releasing Hormone (CRH) compounds 4-(1-ethyl-propoxy)-3,6-dimethyl-2-(2,4,6-trimethyl-phenoxy)-pyridine and (3,6-dimethyl-2-(2,4,6-trimethyl-phenoxy)-pyridin-4-yl)-(1-ethyl-propyl)-amine for the treatment of heart failure.

Further, in EP 0 995 439, a substituted indazole derivative, which inhibits phosphodiesterase type IV and the production of tumor necrosis factor is proposed for the treatment of congestive heat failure.

In spite of a number of different compounds available for treating CHF diuretics and digoxin are still the most utilized agents, which in fact may be due to their capacity to provide effective symptomatic relief. Yet, their use, specifically that of digoxin, is limited because of its slow onset of action and the small difference between the maximum therapeutic and minimum toxic dose levels.

Thus, the discovery and the development of new drugs for treating and/or preventing diseases of the cardiovascular system in humans and animals and in particular of new drugs against heart failure is major desire. For this purpose, the pharmaceutical industry is constantly seeking for molecules displaying promising properties which may serve as new drugs.

Several screening methods for compounds preventing or treating cardiovascular diseases are already known. In this respect, Numann et al. describe a method, wherein compounds may be tested in cell cultures with respect to their ion channel binding properties (Numann and Negulescu, Trends Cardiovasc. Med. 11:54-59 (2001)). According to another method, compounds are tested on isolated and perfused hearts (R. Bessho, D.J.J. Chambers, Thorac Cardiovasc. Surg. 122:993-1003 (2001)). In addition thereto, *in vivo* testing methods are known, wherein the effect of compounds on the cardiovascular system is monitored by the means of electrocardiography, magnetic resonance imaging, or echocardiography in living animals (Chu et al., BMC Physiol 1:6-11 (2001); Krupnick et al., J Heart Lung Transplant 21:233-43 (2002)).

The information resulting from tests on basis of cell cultures is however limited to the cellular level and does not appropriately reflect the situation in the whole organ or organism. On the other hand, the *in vivo* methods of the state of the art provide a more relevant information, but are expensive to perform and have not yet been successfully applied to a High Throughput Screening (HTS) programme.

Thus, an object of the present invention is to provide a novel screening method for the identification of potential drugs for treating or preventing diseases of the cardiovascular system in humans and animals, which is closely simulating the biological situation and may serve as a basis for a high throughput screening.

According to the present invention, this problem is solved by providing a method for the identification of a compound or a composition being a potential drug for treating or preventing diseases of the cardiovascular system of humans and animals, which method comprises culturing a living avian embryo, incubating said avian embryo with at least one first compound or composition containing the same, said at least one first compound or composition causing a detectable change in the activity of the cardiovascular system of said avian embryo, incubating said avian embryo with at least one second compound or composition, said at least one second compound or composition being to be tested as a potential drug, and detecting and/or monitoring an effect of said at least one second compound or composition and/or said at least one second compound or composition on the cardiovascular system of said avian embryo.

The avian embryo represents a well-designed *in vivo* test system. In particular, the limited needs of the bird embryo for a proper development, constrained to an adequate temperature and humidity level, provide many advantages. In addition thereto, the development of the avian embryo is extra-uterine and takes place on the surface of the yolk, providing an easy access for monitoring steps. Furthermore, the heart of the bird embryo is very early functional in the development.

The application of said first and second compounds or compositions may either be performed on the avian embryo itself, on the membrane covering the embryo, on the chorio-allantoic membrane or in the yolk. Preferably, the substances will be applied topically on the embryo itself. The substances may be applied either directly or in form of solutions or suspensions. Advantageously, substances assisting during the passage of said first and second compounds or compositions via the membrane and skin into the organism, such as dimethylsulfoxide (DMSO) are used as a basis for said solutions or suspensions.

Preferably, during the realization of the method of the present invention, said at least one first compound or composition is applied before or simultaneously with said at least one second compound or composition. The results obtained when using the before-mentioned order of application showed to be highly reliable and reproducible. In particular, this order of application provides a very close simulation of the situation after the occurring of cardiovascular health problem in the human body, as will also be outlined in more detail below. In cases of heart failure in humans, a compensating mechanism leads to a permanent adrenaline over-stimulation of the pumping function of the failing heart. This overdrive triggers to desensitization and thus to a drastic reduction of the stroke volume. In such cases, some cardiovascular drugs like β-adrenoreceptor antagonists are used to treat this pre-existing adrenoreceptor over-stimulation (Sabbah H.N. Clin. Cardiol. 22 (1999) V16-20).

The at least one first compound or composition may be selected from various compounds or compositions of the state of the art known to cause a detectable change in the activity of the cardiovascular system, such as e.g. a decrease or increase of the heart contraction ability and/or an alteration of the stroke volume and/or an alteration of the heart beat rhythm and/or an alteration of the heart rate and/or an alteration of the cardiac output.

Preferably, said change in the activity of the cardiovascular system will be effected by the application of a substance stimulating specific receptors, such as an adrenoreceptor agonist, preferably a 13-adrenoreceptor agonist, such as e.g. isoproterenol or noradrenalin, an adenosine receptor agonist, such as e.g. R(-)N6-(2-phenylisopropyl)-adenosine or an acetylcholine receptor agonist, such as e.g acetylcholine. In particular, any compound or composition leading to a so-called desensitization (Bristow M.R., Lancet 352 (1998), 18-14), for example a β-adrenergic desensitization may be used as a first compound or composition according to the present invention.

The at least one second compound or composition is the compound or composition to be tested. In general any compound or composition being a potential drug for treating or preventing diseases of the cardiovascular system of humans or animals, may be utilized.

In particular, the method of the present invention may be used for testing potential drugs having an effect against heart failure in human and/or animals, such as e.g. adrenoreceptor antagonists, preferably β-adrenoreceptor antagonists (so called "beta blockers"), calcium channels antagonists, calcium channel blockers, calcium sensitizers, phosphodiesterase inhibitor etc..

The method of the present invention is not limited to a specific bird species. Preferably, the avian embryo is an embryo of chicken (*Gallus gallus*), quail (*Coturnix coturnix*) or duck (*Anas sp*.).

The bird embryo can be grown *in vitro,* i.e. without the shell. According to one embodiment egg white is essentially separated from egg yolk of a fertilized avian egg, the egg yolk sac, optionally already including the embryo and extra-embryonic structures such as the amnion and allantois, is introduced in a yolk sac container which dimensioned such as to contain the yolk sac without substantial distortion thereof, and said embryo is incubated under *in vitro* culture conditions. Such *in vitro* culturing enables an easy monitoring of the young chick embryo and facilitates an optical monitoring of the heart functionality.

Advantageously, the avian embryo which is used for the method of the present invention is 3-6 days old. However, a skilled person may also choose older or younger embryos for specific applications.

The effects of the incubation of the avian embryo with said at least one second compound or composition and/or said at least one first compound or composition on the cardiovascular system of said bird embryo may be monitored and/or detected by any physical or medical method known in the state of the art, such as e.g. electrocardiography, impedance-cardiography, pulse oximetry, ballistography, accoustography, magnetic resonance imaging and/or an optical method, in particular by a camera. Advantageously, also combinations of said methods may be applied.

The detection and/or monitoring steps will be performed at least after the incubation with said at least one second compound or composition. When appropriate, said detection or monitoring steps may also be realized before the start of the test with the untreated avian embryo and/or after the application of the first compound or composition. The specific choice and combination of the required detection and monitoring steps may easily be performed by a skilled person in view of the particular screening to be realized. For example, for providing further information and for allowing a control of the tests, also the effect of each of the used compounds or compositions or of the solvents alone, etc. may be detected or monitored.

The steps of the method of the present invention may also be realized fully or partially automated, permitting the realization of high throughput screening (HTS) programs.

The method of the present invention provides a method, which may be quickly and at low costs realized. This method may in particular be used for a screening of a library of molecules or of extracts, e.g. of various plants, mushrooms, bacteria, etc. or may serve for the identification of novel leading structures for the development of drugs.

The method of the present invention is closely simulating the human or animal disease mechanisms, as it is an *in vivo* method providing information with respect to the relevant organ, the heart. For example, during a so-called "heart failure", the pumping power of the heart is decreased and the heart cannot fulfil its function properly. This situation may be simulated by incubating a living bird embryo with a high concentration of a beta-receptor agonist, leading to a strong decrease of the heart contractility and thus to a decrease of the stroke volume, i.e. the blood volume ejected from the heart at each heart beat. This state is very similar to the state of the human failing heart, in which an adrenergic overdrive is often noticed. The molecular cause of this decrease of the contractility is probably a decrease in the sensitivity of the adrenoreceptor toward agonists, a phenomenon called desensitization. A protection of the heart led by the compound to be tested thus indicates that the so-called compound displays potentialities against heart failure in humans and animals and thus can be used as a drug or as a leading structure to develop a drug.

The following example illustrates the invention in a more detailed manner. It has, however, to be understood that the present invention is not limited to the example but is rather embraced by the scope of the claims.

### Example

### Decrease of the stroke volume by isoproterenol in the chick embryo and rescue by metoprolol

Fertilized Bovans Goldline brown chicken eggs (12 hrs after laying, kept at 18°C) were incubated at 38°C and 45 % humidity during 72 hrs (Motorbruter 126-EM, Bruja). The eggshell was then broken and the egg white, the yolk and the embryo were removed and placed in a Petri dish (Greiner, 9.4 cm diameter). This Petri dish was placed in a bigger Petri dish (Greiner, 14 cm diameter) containing water and an incubation is performed at 38°C and 45 % humidity for 24 hrs or 48 hrs. The relative stroke volume of the heart of the chick embryo was then estimated by video microscopy using a binocular (SZX-ZB9, Olympus), a CCD camera (F-View, Soft Imaging Systems) and an image analysis software (AnalySIS Auto Software, Soft Imaging Systems). The beating heart of the embryo was filmed and the grey density of the heart area under systole was subtracted from the density of the heart area under diastole. By performing said subtraction, the so-called "relative stroke volume" is obtained, which represents a relative value instead of a volume.

50 µL of a isoproterenol (Sigma) solution (1mM, water) was then topically applied on the surface of the embryo, followed by 20 µL metoprolol (Sigma) solution (15 mM, Vol-90 % water, 10 % dimethylsulfoxide). Metoprolol is a marketed drug for human use against heart failure (M. Andrejak and M. Slama, Presse Med. 27:2025-30 (1998)). After 2 minutes at 22°C, the heart was monitored and the relative stroke volume was measured. The same experiment was performed by replacing the metoprolol solution by a water-dimethylsulfoxide mix (90 %-10 %) in order to investigate the effect of isoproterenol alone. Controls were performed with untreated embryos. The results are shown in table 1 and 2. The test groups of the 96 hrs old embryos (Table 1) comprise seven embryos each, while the test groups of the 120 hrs old embryos (Table 2) comprise five embryos each.

**Table 1**

| Effect of isoproterenol alone or in combination with metoprolol on the relative stroke volume on the 96 hrs old embryos (n=7) | |
|---|---|
| Treatment | % remaining stroke volume |
| Isoproterenol alone | 25,5 |
| Isoproterenol with metoprolol | 57,0 |

**Table 2**

| Effect of isoproterenol alone or in combination with metoprolol on the relative stroke volume on the 120 hrs old embryos (n=5) | |
|---|---|
| Treatment | % remaining stroke volume |
| Isoproterenol alone | 27,2 |
| Isoproterenol with metoprolol | 58,8 |

As may be derived from Table 1 and 2 above, isoproterenol induces a 75 % reduction of the stroke volume after 2 minutes with respect to the 96 hrs old embryos and a 72 % reduction of the stroke volume after 2 minutes incubation with respect to the 120 hrs old embryos.

When applied with metoprolol, this decrease was only 43 % with respect to the 96 hrs old embryos and 41 % with respect to the 120 hrs old embryos.

The method of the present invention allows thus an identification of a compound - the beta-adrenoreceptor antagonist metoprolol - conferring a protection to the heart against the drop of the stroke volume induced by another compound - isoproterenol.

## Claims

1. A method for identifying a compound or a composition being a potential drug for treating or preventing diseases of the cardiovascular system of humans and animals, comprising:
culturing a living avian embryo;
incubating said avian embryo with at least one first compound or composition, said at least one first compound or composition causing a detectable change in the activity of the cardiovascular system of said avian embryo;
incubating said avian embryo with at least one second compound or composition, said at least one second compound or composition being to be tested as a potential drug; and
detecting and/or monitoring an effect of said at least one second compound or composition and/or said at least one first compound or composition on the cardiovascular system of said avian embryo.

2. The method according to claim 1, wherein said at least one first compound or composition is causing a decrease or increase of the heart contractility and/or an alteration of the stroke volume and/or an alteration of the heart beat rhythm and/or an alteration of the heart rate and/or an alteration of the cardiac output.

3. The method according to claim 2, wherein said at least one first compound or composition is an adrenoreceptor agonist, preferably a beta adrenoreceptor agonist.

4. The method according to claim 1, wherein said at least one second compound or composition is to be tested with respect to an effect against heart failure in human and/or animals.

5. The method according to claim 4, wherein said at least one second compound or composition is a adrenoreceptor antagonist, preferably a beta adrenoreceptor antagonist.

6. The method according to claim 1, wherein the bird is a chicken (Gallus gallus), a quail (Coturnix coturnix) or a duck (Anas sp.).

7. The method according to claim 1, wherein said avian embryo is *in vitro* or *in ovo* cultured.

8. The method according to claim 1, wherein said avian embryo is 3-6 days old.

9. The method according to claim 1, wherein said step of detecting and/or monitoring an effect on the cardiovascular system of said bird embryo involves a physical or medical method, preferably electrocardiography, impedance-cardiography, acoustography, ballistography, pulse oximetry, an optical method and/or magnetic resonance imaging.

10. The method according to claim 1, wherein the process steps are fully or partially automated.
